(19) Europäisches Patentamt
European Patent Office
Office européen des brevets

(11) Veröffentlichungsnummer: **0 205 889 B1**

# (12) EUROPÄISCHE PATENTSCHRIFT

(45) Veröffentlichungstag der Patentschrift: **15.07.92**

(51) Int. Cl.[5]: **C08G 77/52**, C08G 77/382, C12N 11/00

(21) Anmeldenummer: **86106592.8**

(22) Anmeldetag: **15.05.86**

## (54) Funktionalisierte Phenylengruppen-haltige Organopolysiloxane und Verfahren zu ihrer Herstellung.

(30) Priorität: **25.05.85 DE 3518880**

(43) Veröffentlichungstag der Anmeldung:
**30.12.86 Patentblatt 86/52**

(45) Bekanntmachung des Hinweises auf die Patenterteilung:
**15.07.92 Patentblatt 92/29**

(84) Benannte Vertragsstaaten:
**AT BE CH DE FR GB IT LI NL SE**

(56) Entgegenhaltungen:
**DE-B- 2 758 507**
**GB-A- 1 442 067**

(73) Patentinhaber: **Degussa Aktiengesellschaft**
**Weissfrauenstrasse 9**
**W-6000 Frankfurt am Main 1(DE)**

(72) Erfinder: **Panster, Peter, Dr.**
**Im Lochseif 8**
**W-6458 Rodenbach(DE)**
Erfinder: **Kleinschmit, Peter, Dr.**
**Wildaustrasse 19**
**W-6450 Hanau 9(DE)**

**EP 0 205 889 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind neue Phenylengruppen-haltige Organopolysiloxane, die durch eine geeignete Funktionalisierung als äußerst effektive Träger von Wirkstoffen Verwendung finden können. Diese neuen Träger besitzen einen hohen Grad an Funktionalisierbarkeit und darüber hinaus die hervorragenden Eigenschaften anorganischer Trägermaterialien.

Ein weiterer Gegenstand der Erfindung ist ein Verfahren zur Herstellung dieser funktionalisierten Organopolysiloxane. Verfahren zur Herstellung ihrer monomeren Vorstufen sowie die monomeren Vorstufen selbst werden beschrieben.

Wirkstoffe oder funktionelle Gruppen, die über chemische Bindungen an einen unlöslichen Träger gebunden sind, besitzen beim technischen Einsatz gegenüber in homogener Phase eingesetzten Wirkstoffen oder funktionellen Gruppen die Vorteile einer leichten Abtrennbarkeit, Recyclierbarkeit und Rückgewinnbarkeit der aktiven Komponenten.

Darüber hinaus können Stabilität und Standzeit eines nach diesem Prinzip modifizierten Agens oft deutlich erhöht und seine Selektivität in einem gewünschten Sinne beeinflußt werden. Während z. B. Ionenaustauscher bereits klassische Beispiele für dieses Konzept darstellen, sind z. B. Trägerfixierte Enzyme oder Metallkomplexkatalysatoren Gegenstand jüngerer Untersuchungen und Synthesebemühungen.

Als Träger eingesetzt wurden für diesen Zweck bisher überwiegend organische Polymere, insbesondere Polystyrol. Beispiele hierfür werden z. B. in der britischen Patentschrift 1 277 736 oder in der US-Patentschrift 3 708 462 gegeben. Obwohl anorganische Polymersysteme, wie z. B. Kieselsäure oder Kieselgel, eine Reihe von Vorteilen besitzen, sind sie im allgemeinen für diese Verwendung weniger geeignet, denn sie sind nur in geringem Umfang funktionalisierbar und die funktionelle Einheit kann hydrolytisch relativ leicht abgespalten werden. In Ermangelung wirklich geeigneter Trägersysteme wurde auch schon versucht, Phenylsiloxane auf Silicagel zu verankern (vgl. J. Conan, M. Bartholin und A. Guyet, J. Mol. Catal. 1, 375 (1975/76) ). Derartige Systeme besitzen im Prinzip aber dieselben Nachteile wie reine anorganische Träger selbst.

Aufgabe der vorliegenden Erfindung war es daher, Trägersysteme bereitzustellen, die sowohl die Vorteile der organischen als auch die der anorganischen Trägermaterialien in sich vereinigen, d. h. daß sie eine festliegende starre Struktur, hohe Temperatur- sowie Alterungsbeständigkeit besitzen, daß sie nur wenig oder gar nicht quellbar und unlöslich in organischen Lösungsmitteln sind und eine hohe Kapazität für Wirkstoffe oder funktionelle Gruppen aufweisen, die leicht zugänglich und fest im Polymergerüst verankert werden können.

Die Aufgabe wurde mit Hilfe neuer Phenylengruppen-haltiger Organopolysiloxane, die nach Stoff und Herstellung in der mit gleichem Einreichungsdatum hinterlegten Parallelanmeldung EP 0 205 890 offenbart ist, gelöst. Sie sind nach gängigen Konzepten der organischen Synthese entweder aus den genannten Polysiloxanen oder aus entsprechenden monomeren Vorprodukten zugänglich. Diese funktionalisierten Phenylengruppen-haltigen Organopolysiloxane sind dadurch gekennzeichnet, daß sie Einheiten der Formel

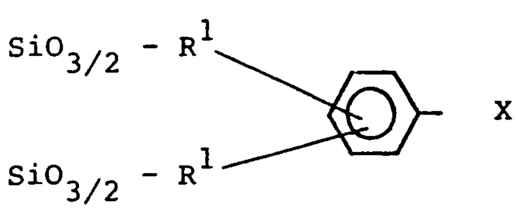

(1)

enthalten, in der jeweils alle 3 denkbaren Isomeren in bezug auf die Stellung der $SiO_{3/2}$-$R^1$-Substituenten an der Phenylengruppe nebeneinander vorliegen können, wobei die Brückenglieder $R^1$ für die Gruppen $-CH_2-CH_2-$ oder $CH_3CH<$ stehen und gleich oder verschieden sein können,
und X für Cl, Br, $CH_2Cl$, $P(C_6H_5)_2$, $CH_2P(C_6H_5)_2$ steht, und die freien Valenzen der Sauerstoffatome durch Siliciumatome gleicher oder verschiedener Gruppen der Formel (1) und/oder durch vernetzende Brückenglieder

$SiO_{4/2}$ oder $R'SiO_{3/2}$ oder $R'_2\,SiO_{2/2}$,
$TiO_{4/2}$ oder $R'TiO_{3/2}$ oder $R'_2\,TiO_{2/2}$,
$ZrO_{4/2}$ oder $R'ZrO_{3/2}$ oder $R'_2\,ZrO_{2/2}$,
$AlO_{3/2}$ oder $R'AlO_{2/2}$

wobei R' eine Methyl- oder Ethylgruppe ist, und/oder durch Phenyleneinheiten der allgemeinen Formel

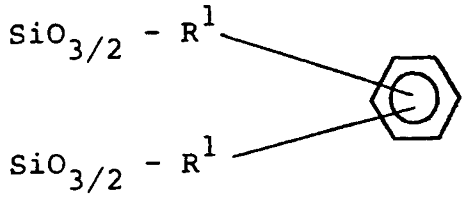
(2)

in der die Brückenglieder $R^1$ denselben Bedeutungsumfang wie bei Formel (1) haben und gleich oder verschieden sein können, abgesättigt sind und das Verhältnis der Summe der Si-Atome in Formel (1) und (2) zu den Brückenatomen Silicium, Titan, Zirkonium und Aluminium 1 : 0 bis 1 : 15 betragen kann.

Das Verhältnis der den Substituenten X tragenden Phenyleneinheiten nach Formel (1) zu den im Polymerverband gegebenenfalls vorhandenen Si-, Ti-, Zr- und Al-haltigen Vernetzergruppen und den Phenylengruppen nach Formel (2) wird nach unten dadurch begrenzt, daß eine Mindestkonzentration von 0,01 meq an Gruppen X/g Organopolysiloxan und eine Höchstkonzentration dann gegeben ist, wenn keine unsubstituierten Phenylengruppen nach Formel (2) und keine vernetzenden Si-, Ti-, Zr- oder Al-haltigen Brückenglieder im Feststoff vorhanden sind. Diese Situation muß jedoch auch in diesem Fall nicht unbedingt gegeben sein, denn je nach Verwendung dieser Polymeren mit Substituenten X tragenden Einheiten nach Formel (1) kann es z. B. zum Zwecke der Steuerung der Dichte der Gruppen X oder zur Einstellung bestimmter spezifischer Oberflächen oder Oberflächeneigenschaften usw. von Vorteil sein, wenn vernetzende Brückenglieder der genannten Art oder auch Gruppen nach Formel (2) im Feststoff vorhanden sind. So kann es z. B. von Vorteil sein, wenn der polymere Feststoff überwiegend nur an der Oberfläche Einheiten der Formel (1) besitzt, so daß bei Folgereaktionen oder direktem Einsatz keine Diffusionsprobleme für die anderen Reaktionspartner auftreten.

Ein weiterer Gegenstand der Erfindung ist das Verfahren zur Herstellung der funktionalisierte Phenylengruppen der Formel (1) enthaltenden Organopolysiloxane. Das Verfahren ist dadurch gekennzeichnet, daß man ein Phenylengruppenhaltiges Organopolysiloyan, welches gleiche oder verschiedene Einheiten der allgemeinen Formel

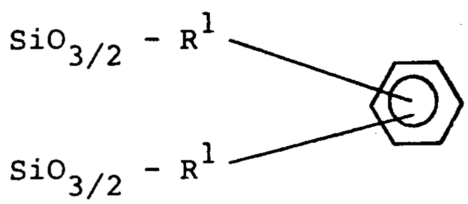
(2)

enthält, in der jeweils alle 3 denkbaren Isomeren in bezug auf die Stellung der $SiO_{3/2}$-$R^1$-Substituenten an der Phenylengruppe nebeneinander vorliegen können, wobei die Brükkenglieder $R^1$ für die Gruppen $-CH_2-CH_2-$ oder $CH_3-CH\langle$ stehen und gleich oder verschieden sein können, und die freien Valenzen der Sauerstoffatome durch Siliciumatome weiterer Gruppen der Formel (2) und/oder durch vernetzende Brükkenglieder

$SiO_{4/2}$ oder $R'SiO_{3/2}$ oder $R'_2\ SiO_{2/2}$,
$TiO_{4/2}$ oder $R'TiO_{3/2}$ oder $R'_2\ TiO_{2/2}$,
$ZrO_{4/2}$ oder $R'ZrO_{3/2}$ oder $R'_2\ ZrO_{2/2}$,
$AlO_{3/2}$ oder $R'AlO_{2/2}$

wobei R' eine Methyl- oder Ethylgruppe ist, abgesättigt sind und das Verhältnis der Summe der Si-Atome in Formel (2) zu den Brückenatomen Silicium, Titan, Zirkonium und Aluminium 1 : 0 bis 1 : 15 betragen kann, in einem Lösungsmittel, bei Temperaturen von -80° C bis 200° C, bei Normaldruck oder einem Überdruck, welcher der Summe der Partialdrucke bei der jeweiligen Temperatur entspricht, mit stöchiometrischen, unterschüssigen oder überschüssigen Mengen eines teilweise oder vollständig gelösten gasförmigen, flüssigen oder festen Reagenzes für die an sich bekannte Substitution (mindestens) eines ringständigen Wasserstoffatoms durch den für eine beabsichtigte Anwendung erforderlichen funktionellen Substituenten X über einen Zeitraum von wenigen Minuten bis zu mehreren Tagen bei Temperaturen von -78° C bis 200° C umsetzt, diesen Umsetzungsvorgang gegebenenfalls nach einem Wechsel des Lösungsmittels wiederholt, anschließend den Feststoff von der flüssigen Phase nach gängigen Techniken abtrennt oder das

Lösungsmittel destillativ entfernt, den modifizierten Feststoff gegebenenfalls unter Verwendung eines anderen Lösungsmittels wäscht, dann gegebenenfalls unter Schutzgasatmosphäre oder unter Verwendung von Vakuum bis zu einer Temperatur von 200° C trocknet, anschließend gegebenenfalls 1 Stunde bis zu 5 Tage bei Temperaturen von 100 - 400° C an der Luft oder unter Schutzgas, bei Normaldruck, unter Vakuum oder Überdruck tempert, gegebenenfalls mahlt und/oder klassifiziert, wobei verschiedene dieser Maßnahmen unterbleiben oder in einer anderen Reihenfolge durchgeführt werden können.

Die als Ausgangsmaterial verwendeten Phenylengruppen-haltigen Organopolysiloxane der Formel (2) können dadurch erhalten werden, daß ein Silan der allgemeinen Formel

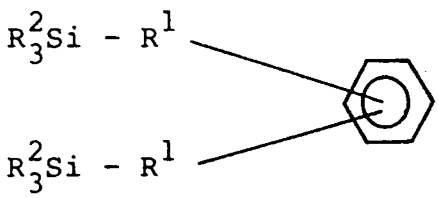

(3)

in der die Brückenglieder $R^1$ für die Gruppen $-CH_2-CH_2-$oder $CH_3-CH<$ stehen und gleich oder verschieden sein können, und die Substituenten $R^2$ für einen linearen oder verzweigten Alkoxirest mit 1 bis 3 C-Atomen oder für Chlorid stehen und gleich oder verschieden sein können, gegebenenfalls nach Zusatz eines Lösungsmittels und/oder einer Vernetzervorstufe der allgemeinen Formel

$$MeR^3_{2-4}R'_{0-2} \quad \text{bzw.} \quad MeR^3_{2-3}R'_{0-1}$$

wobei Me = Si, Ti, Zr bzw. Al
$R^3$ für einen linearen oder verzweigten Alkoxirest mit 1 - 5 C-Atomen oder für Chlorid steht und
R' eine Methyl- oder Ethylgruppe ist, mit stöchiometrischen oder überschüssigen Mengen Wasser hydrolysiert und polykondensiert, das Produkt gegebenenfalls nach Zusatz weiteren Lösungsmittels von der flüssigen Phase abgetrennt, gegebenenfalls unter Schutzgasatmosphäre oder unter Verwendung von Vakuum bis zu einer Temperatur von 200° C getrocknet, anschließend gegebenenfalls 1 Stunde bis zu 5 Tagen bei Temperaturen von 100 - 400° C an der Luft oder unter Schutzgas, bei Normaldruck, unter Vakuum oder Überdruck getempert, gegebenenfalls gemahlen und klassifiziert wird.

Bezüglich der Stabilität der neuen Phenylengruppen-haltigen Organopolysiloxane gegenüber Art oder Auflösen bei erhöhter Temperatur in Wasser oder aggressiven polaren organischen Solvenzien ist es von Vorteil,wenn das Produkt nach seiner Herstellung, gegebenenfalls in Verbindung mit einer Trocknung oder auch erst vor seiner Verwendung der erwähnten Temperung ausgesetzt wird. Die Maßnahme der Temperung ist von der Synthese von anorganischen Polymeren, wie z. B. Kieselsäuren oder Kieselgelen, her bekannt. Sie bewirkt eine weitere Dehydratisierung bei Reaktion benachbarter Silanolgruppen oder eine Abspaltung von noch in der polymeren Substanz vorhandenen Alkoxigruppen bzw. Si-gebundenen Chloratomen in Gestalt des entsprechenden Alkohols bzw. von Chlorwasserstoff bei gleichzeitiger Ausbildung von Siloxanbindungen.

Im Prinzip könnte $R^2$ noch für weitere Substituenten, wie z. B. Br, J, $OC_6H_5$ oder $OC_2H_4OCH_3$, stehen, doch bietet deren Verwendung keine Vorteile, sondern eher Nachteile, z. B. hinsichtlich der Zugänglichkeit der entsprechenden Silane oder in bezug auf die Hydrolysegeschwindigkeit und die bei der Hydrolyse anfallenden Nebenprodukte. Verschiedentlich, z. T. in Abhängigkeit von der Art des verwendeten Lösungsvermittlers und wenn $R^2$ für einen linearen oder verzweigten Alkoxirest steht, kann es von Vorteil sein, dem zu polykondensierenden Silan eine kleine Menge eines der üblichen Polykondensationskatalysatoren, im einfachsten Fall wäßrige HCl-Lösung, zuzusetzen. Unter diesem Aspekt betrachtet, ist verständlicherweise die Hydrolysegeschwindigkeit am größten, wenn $R^2$ für Chlorid steht.

Obwohl die Hydrolyse und Polykondensation auch ohne Verwendung eines Lösungsvermittlers durchführbar ist, ist eine Benutzung aus praktischen Gründen meist vorzuziehen.

Die Wahl des Lösungsmittels bei der Einführung des Substituenten X richtet sich in erster Linie nach den Reagenzien, mit denen die als Ausgangsmaterial verwendeten Organopolysiloxane nach Formel (2) zur Umsetzung gebracht werden. Da es sich dabei um gängige Lehrbuch-bekannte Reaktionsprinzipien der

organischen Synthese handelt, sind diese Lösungsmittel generell auch dieselben, die bei entsprechenden Reaktionen mit Monomeren verwendet werden. Sie bedürfen daher keiner weiteren Aufzählung.

Bei der Umsetzung von Feststoffen mit gelösten, festen oder gasförmigen Reaktionspartnern ist es aus Gründen der begünstigten Diffusion generell von Vorteil, wenn der jeweilige Feststoff relativ feinteilig ist. Es kann daher sinnvoll sein, diesen vor oder auch während der Umsetzung mit dem Funktionalisierungsagens zu mahlen. Alternativ kann es natürlich im Hinblick auf bestimmte Anwendungen der funktionalisierten Phenylengruppen-haltigen Polysiloxane, z. B. in einem Festbettreaktor, von Vorteil sein, den Feststoff in möglichst grobkörniger Form zu belassen. In diesem Fall kann nach einer Variante des erfindungsgemäßen Funktionalisierungsverfahrens z. B. auch nur der Oberflächenbereich eines Feststoffpartikels des Organopolysiloxans nach Formel (2) chemisch in der genannten Art und Weise modifiziert werden. Dies kann z. B. durch Verwendung unterstöchiometrischer Mengen an Funktionalisierungsagens oder durch kurze Umsetzungszeiten erreicht werden.

Geeignete Funktionalisierungsagentien, mit deren Hilfe eine Funktionalisierung der Polymeren, bestehend aus Einheiten der Formel (2), erreicht werden kann, sind insbesondere elementares Chlor oder Brom, Chlormethylmethyl- oder Chlormethylethylether, Formaldehyd, Paraformaldehyd, das Methyl- oder Ethylacetal von Formaldehyd plus Chlorwasserstoff und Lithium-, Natrium- oder Kaliumdiphenylphosphid. So kann die Chlorierung oder Bromierung dieser Polymeren durch Umsetzung mit Chlor oder Brom in einem Lösungsmittel wie Tetrachlorkohlenstoff in Gegenwart von Eisenpulver oder Eisen(III)-chlorid oder anderen Lewis-Säuren erfolgen (vgl. z. B. G.B. Bachman et al., J. Org. Chem. 12, 108 (1947) ). Die Chlormethylierung kann mit Hilfe von Chlormethylmethyl- oder Chlormethylethylether, in Gegenwart von wasserfreiem Zinntetrachlorid in Chloroform oder durch Reaktion mit Formaldehyd oder Modifikation davon und Chlorwasserstoffgas durchgeführt werden (vgl. z. B. R.B. Merrifield, J. Amer. Chem. Soc. 85, 2149 (1963) ). Die Einführung der Diphenylphosphino-Gruppe an dem vorher halogenierten oder chlormethylierten Polymer kann z. B. wie durch K.G. Allum et al. in J. Organometal. Chem. 87, 189 (1975) beschrieben, erfolgen.

Um bei obengenannten Modifizierungen eventuell denkbare Nebenreaktionen an der Polysiloxan-Matrix weitgehend auszuschalten, kann es von Vorteil sein, den zu modifizierenden Feststoff gemäß Formel (2) vorher einer Temperbehandlung der beschriebenen Art zu unterziehen. Alternativ kann ein analoger Effekt, d. h. Abreaktion noch vorhandener Rest-Silanolgruppen, z. B. auch durch Umsetzung mit $ClSi(CH_3)_3$ erzielt werden, wie dies z. B. in J. Organometal. Chem. 87, 189 (1975) beschrieben ist.

Alternativ zu vorstehend beschriebenen Verfahren zur Herstellung von Polysiloxanen, die funktionalisierte Einheiten der Formel (1) enthalten, können im Prinzip auch die monomeren Silane gemäß Formel (3) in der genannten Weise funktionalisiert werden. Obwohl dies sicher bei einzelnen Reaktionstypen praktikabel ist, ist in der Regel, insbesondere wegen der in diesen Fällen gegebenen zusätzlichen Reaktivität an den Alkoxisilyl- bzw. Chlorsilylgruppen die Funktionalisierung der Polymeren mit Einheiten der Formel (2) vorzuziehen.

Die erfindungsgemäßen Organopolysiloxane besitzen je nach Ausgangsmaterial, verwendetem Polykondensationsmedium und Polykondensationsbedingungen spezifische Oberflächen von weniger als 1 $m^2/g$ bis zu 1000 $m^2/g$. Die Teilchengrößen können innerhalb bestimmter Bereiche eingestellt werden; sie liegen zwischen ca. 0,1 m bis zu 1 cm.

Die Erfindung wird nachfolgend anhand von Ausführungsbeispielen unter Verwendung der wichtigsten Ausgangsmaterialien näher erläutert. Dabei betreffen die Beispiele 1 bis 3 die Herstellung von Zwischen produkten für die in den Beispielen 4 bis 9 beschriebene Herstellung der anmeldungs gemäßen Organopolysiloxane.

## Beispiel 1

100 g einer isomeren Verbindung, bestehend zu ca. 90 Gew.% aus dem ortho-, meta-, para-Isomerengemisch (12 Gew.% / 65 Gew.% / 23 Gew.%) des Chlorsilans

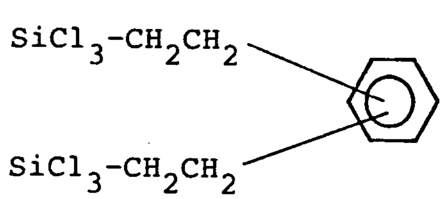

und zu ca. 10 % aus den Chlorsilanen

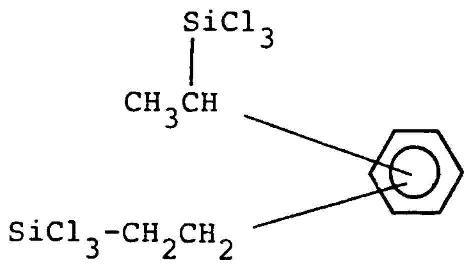

und

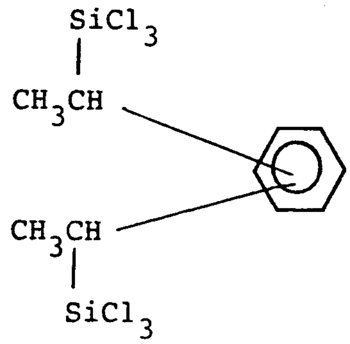

(gewichtsmäßige Mengenverteilung 2 : 1)
mit der gleichen Isomerenverteilung wurden in rund 100 ml Toluol gelöst. Die Lösung wurde in einem 1 1-Dreihalskolben mit KPG-Rührer und Rückflußkühler unter kräftigem Rühren und zunächst unter Eiskühlung innerhalb von 30 min. mit 100 g entsalztem Wasser versetzt. Unter starkem Schäumen setzte bereits nach Zusatz von 30 ml $H_2O$ eine spontane Verdickung ein, so daß der Kolbeninhalt kurzfristig nicht mehr rührfähig war. Nach Zugabe von weiteren 50 ml Toluol und 50 ml Wasser wurde auf Rückflußtemperatur erhitzt und 2 Stunden unter Rückfluß gerührt. Anschließend wurde abgekühlt und der gebildete weiße Feststoff über eine Nutsche abfiltriert, zunächst mit 100 ml Ethanol und dann mit 3 l Wasser nahezu HCl-frei gewaschen. Nach 12-stündiger Trocknung bei 150° C/100 mbar und 30-stündiger Temperung bei 250° C unter $N_2$-Atmosphäre konnten 58,8 g (99,8 % der Theorie) des gewünschten Phenylengruppen-haltigen Organopolysiloxans bestehend zu ca. 90 % aus Einheiten der Formel

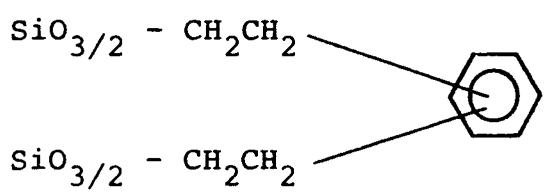

und zu ca. 10 % aus Einheiten der Formel

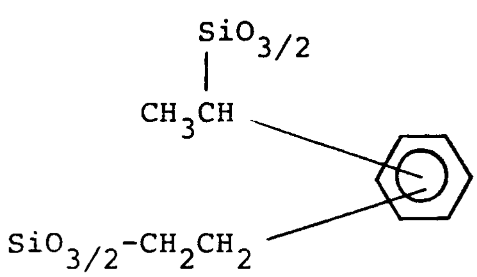

und

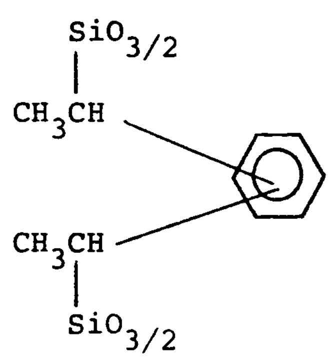

(entsprechend der im Ausgangsmaterial vorgegebenen gewichtsmäßigen Mengenverteilung)
in Form eines weißen Pulvers erhalten werden.

| Elementaranalysen | % C | % H | % Si | % Cl |
|---|---|---|---|---|
| Theorie: | 50,81 | 5,12 | 23,76 | 0 |
| Gefunden: | 48,95 | 5,33 | 22,47 | 0,02 |

Das Produkt wurde nach der Trocknung und Temperung gemahlen und klassifiziert. Von der Korngrößenfraktion 0,3 - 1,2 mm wurde eine Bestimmung der spezifischen Oberfläche am Areameter durchgeführt

die einen Wert von 338 $m^2/g$ ergab. Eine durchgeführte DSC-Untersuchung des Produktes unter $N_2$-Atmosphäre ergabe eine beginnende endotherme Zersetzung des Polymers bei über 280° C.

Beispiel 2

100 g einer isomeren Verbindung, bestehend zu ca. 90 % aus dem ortho-, meta-, para-Isomerengemisch (12 Gew.% / 65 Gew.% / 23 Gew.%) des Ethoxisilans

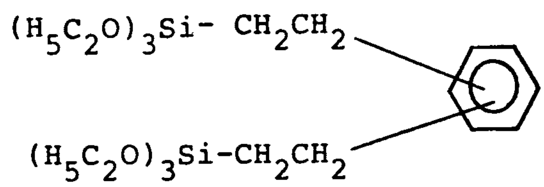

und zu ca. 10 % aus den Ethoxisilanen

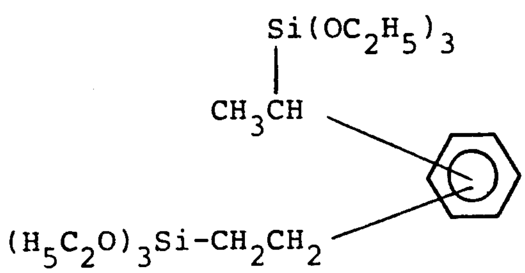

und

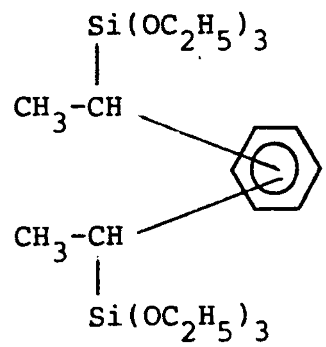

(gewichtsmäßige Mengenverteilung 2 : 1)
mit der gleichen Isomerenverteilung wurden mit 120 ml Ethanol gemischt. Die Mischung wurde in einem 1 l-Dreihalskolben mit KPG-Rührer, Rückflußkühler und Tropftrichter auf Rückflußtemperatur aufgeheizt und unter kräftigem Rühren auf einmal mit 50 ml $H_2O$ versetzt.

Schon wenige Minuten nach der Wasserzugabe verdickte sich der Ansatz und es entstand ein voluminöser Feststoff. Dieser wurde noch 2 Stunden unter Rückfluß gerührt, dann über ein Saugfilter abfiltriert und erst mit 100 ml Ethanol und dann mit 2 l $H_2O$ gewaschen. Nach 24-stündiger Trocknung bei 150° C/100 mbar wurden 52,7 g (102,3 % der Theorie) des gewünschten Produkts in Form eines weißen Feststoffs erhalten. Die Zusammensetzung dieses Organopolysiloxans entsprach der des in Beispiel 1 erhaltenen Produkts, sowohl in bezug auf die Struktur als auch hinsichtlich der Isomerenverteilung.

| Elementaranalysen: | % C | % H | % Si |
|---|---|---|---|
| Theorie: | 50,81 | 5,12 | 23,76 |
| Gefunden: | 49,03 | 6,01 | 22,34 |

Die 0,3 - 1,2 mm Fraktion des klassifizierten Produkts besaß eine spezifische Oberfläche von 89 $m^2/g$ (Areameter).

Beispiel 3

75 g des in Beispiel 2 eingesetzten Ausgangsmaterials und 68,12 g $Si(OC_2H_5)_4$ wurden in 100 ml Ethanol vereinigt. Die Mischung wurde in einem 1 1-Dreihalskolben mit KPG-Rührer, Rückflußkühler und Tropftrichter auf Rückflußtemperatur erhitzt. Unter kräftigem Rühren wurden auf einmal 50 9 Wasser zugesetzt. Der Kolbeninhalt gelierte sofort nach Zugabe des Wassers. Der Kolbeninhalt wurde noch eine Stunde unter Rückfluß gerührt, dann abgekühlt, abfiltriert und mit 300 ml Ethanol gewaschen. Nach 10-stündiger Trocknung bei 150° C und 2-stündiger Temperung bei 300° C unter $N_2$-Atmosphäre wurden

58,9 g (101,0 % der Theorie) eines polymeren Produkts, bestehend zu ca. 90 % aus Einheiten der Formel

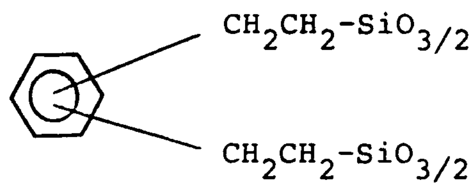 · $2SiO_2$

und zu ca. 10 % aus Einheiten der Formel

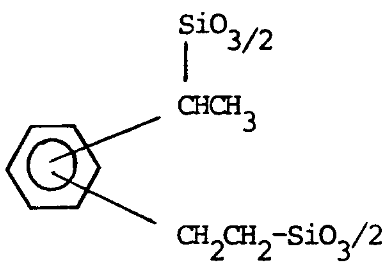 · $2SiO_2$ und 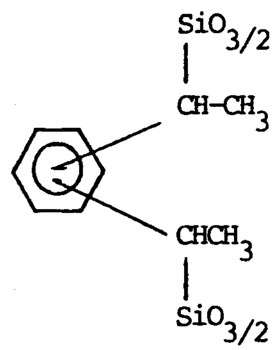 · $2SiO_2$

(entsprechend der im Ausgangsmaterial vorgegebenen gewichtsmäßigen Mengenverteilung) mit einem ortho- / meta- / para-Isomerenverältnis von 12 Gew.% / 65 Gew.% / 23 Gew.% erhalten.

| Elementaranalysen: | % C | % H | % Si |
|---|---|---|---|
| Theorie: | 33,69 | 3,39 | 31,51 |
| Gefunden: | 32,21 | 3,56 | 30,87 |

Beispiel 4

20 g des in Beispiel 1 hergestellten Phenylengruppen-haltigen Organopolysiloxans mit einer eingestellten Korngröße von 0,1 - 0,2 mm wurden zusammen mit 150 mg Eisenpulver in 75 ml Chloroform vereinigt. Bei Raumtemperatur wurden zu dieser Suspension in einem 250 ml Dreihalskolben mit KPG-Rührer, Rückflußkühler und Tropftrichter unter kräftigem Rühren 14,87 g Brom, gelöst in 20 ml Chloroform, während 15 min. zugetropft. Anschließend wurde die Suspension innerhalb 1 Stunde auf Rückflußtemperatur aufgeheizt. Es wurde noch 3 Stunden bei dieser Temperatur gerührt, dann abgekühlt und der Feststoff abfiltriert. Dieser wurde zuerst mit 100 ml $CHCl_3$ und dann mit 100 ml Ethanol gewaschen. Nach 14-stündiger Trocknung bei 120° C/100 mbar wurden 24,5 g des gewünschten Produkts mit einem Bromgehalt von 20,33 % erhalten. Bei vollständiger einfacher Bromierung aller im Feststoff vorhandenen Phenylengruppen war ein Bromgehalt von 25,34 % zu erwarten.

Beispiel 5

10 g des in Beispiel 1 hergestellten Phenylengruppen-haltigen Organopolysiloxans mit einer eingestellten Korngröße von 0,3 - 1,2 mm wurden zusammen mit 100 mg wasserfreiem Eisen(III)-chlorid in 75 ml $CCl_4$ vereinigt. In einem 250 ml Dreihalskolben mit Sole-Rückflußkühler (-20° C), KPG-Rührer und Gaseinleitungsrohr wurde bei Raumtemperatur innerhalb von 4 Stunden unter kräftigem Rühren elementares Chlor durch diese Suspension geleitet. Danach wurde der Feststoff abfiltriert mit 200 ml $CCl_4$ gewaschen und 15 Stunden bei 120° C/100 mbar getrocknet. Es konnten 10,3 g des gewünschten Produkts mit einem Chlorgehalt von 6,50 % erhalten werden. Dies entspricht einer rund 50 %igen Monochlorierung aller vorhandenen Phenylengruppen im Polysiloxan.

Beispiel 6

20 g des in Beispiel 2 hergestellten Phenylengruppen-haltigen Organopolysiloxans und 100 ml Petrolether (80 - 110° C) wurden in einem 500 ml Dreihalskolben mit KPG-Rührer, Rückflußkühler und Tropftrichter vereinigt. Bei einer Temperatur von 35 - 40° C wurden zu dieser Suspension ein Gemisch von 25 ml Chlordimethylether und 2,5 ml $SnCl_4$ innerhalb von 30 min. zugetropft. Es wurde noch 4 Stunden bei dieser Temperatur gerührt, dann der Reaktionssuspension 200 ml Eiswasser zugesetzt. Nach weiterem 0,5-stündigem Rühren wurde abfiltriert, der Feststoff mit 150 ml Methanol gewaschen und 10 Stunden bei 130° C/100 mbar getrocknet. Das ausgewogene Produkt (20,3 g) besaß einen Chlorgehalt von 4,0 %, was einer rund 30 %igen Chlormethylierung aller vorhandenen Phenylengruppen entspricht.

Beispiel 7

20 g des in Beispiel 3 hergestellten $SiO_2$-vernetzten Phenylengruppen-haltigen Organopolysiloxans wurden in 100 g konzentrierter Salzsäure suspendiert. In einem 500 ml Dreihalskolben mit KPG-Rührer, Rückflußkühler und Tropftrichter bzw. Gaseinleitungsrohr wurden zu dieser Suspension zunächst bei 50° C 10 g 40 %iger Formalinlösung innerhalb 1 Stunde zugetropft. Anschließend wurde die Suspension noch 5 Stunden bei 50° C gerührt, wobei über das Gaseinleitungsrohr Chlorwasserstoffgas eingeleitet wurde. Nach dieser Zeit wurde abfiltriert, der verbleibende Feststoff mit 300 ml entsalztem Wasser gewaschen und dann 24 Stunden bei 130° C/100 mbar getrocknet. Das ausgewogene Produkt besaß einen Chlorgehalt von 4,8 %, was einer rund 50 %igen Chlormethylierung aller vorhandenen Phenylengruppen entsprach.

Beispiel 8

9 g des in Beispiel 4 hergestellten bromierten Phenylengruppen-haltigen Organopolysiloxans wurden in 40 ml n-Hexan suspendiert. Die Suspension wurde in einem Kühlbad auf -25° C abgekühlt. Unter kräftigem Rühren wurden zu dieser Suspension 17,2 ml einer 1,6 molaren Lithiumbutyl-Lösung in n-Hexan innerhalb von 15 min. zugetropft. Anschließend wurde noch 6 Stunden bei dieser Temperatur gerührt, dann wurde das Lösungsmittel aus dem Kolben herausgesaugt und frisches, auf -25° C gekühltes n-Hexan zugegeben. Danach wurden 5,1 ml $ClP(C_6H_5)_2$, verdünnt mit 5 ml n-Hexan, innerhalb von 10 min. zugetropft. Die Mischung wurde noch 8 Stunden bei Raumtemperatur gerührt, dann wurde der Feststoff abfiltriert, mit 30 ml n-Hexan und dann mit 100 ml Ethanol gewaschen und 3 Stunden bei 150° C/0,1 mbar getrocknet. Es konnten 9,2 g des gewünschten Produkts mit einem Phosphorgehalt von 2,51 % erhalten werden. Dies bedeutet, daß ca. 25 % aller vorhandenen Bromatome durch $P(C_6H_5)_2$-Gruppen ersetzt worden sind.

Beispiel 9

10,0 g des in Beispiel 6 hergestellten chlormethylierten Phenylengruppen-haltigen Organopolysiloxans wurden in 40 ml getrocknetem Tetrahydrofuran suspendiert. Unter Rühren wurde diese Suspension bei Raumtemperatur mit 14,9 ml einer Lösung von $LiP(C_6H_5)_2$ in Tetrahydrofuran (entspricht 3,50g $LiP(C_6H_5)_2$)-versetzt und anschließend 6 Stunden unter Rückfluß gerührt. Es wurde abgekühlt, der Feststoff abfiltriert, mit 50 ml Tetrahydrofuran sowie mit 50 ml Ethanol gewaschen und 3 Stunden bei 150° C/0,1 mbar getrocknet. Die erhaltenen 11,1 g Produkt besaßen einen P-Gehalt von 1,5 %, was einer rund 65 %igen Substitution der vorhandenen Chloratome durch $P(C_6H_5)_2$-Gruppen entspricht.

**Patentansprüche**

1. Funktionalisierte Phenylengruppen-haltige Organopolysiloxane, dadurch gekennzeichnet, daß sie gleiche oder verschiedene Einheiten der Formel

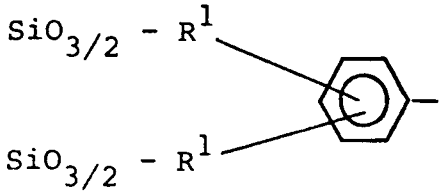

(1)

enthalten, in der jeweils alle 3 denkbaren Isomeren in bezug auf die Stellung der $SiO_{3/2}$-$R^1$-Substituenten an der Phenylengruppe nebeneinander vorliegen können, wobei die Brückenglieder $R^1$ für die Gruppen $-CH_2-CH_2-$oder $CH_3-CH<$ stehen und gleich oder verschieden sein können,
X für Cl, Br, $CH_2Cl$, $P(C_6H_5)_2$, $CH_2P(C_6H_5)_2$ steht und die freien Valenzen der Sauerstoffatome durch Siliciumatome weiterer Gruppen der Formel (1) und/oder durch vernetzende Brückenglieder

$SiO_{4/2}$ oder $R'SiO_{3/2}$ oder $R'_2\ SiO_{2/2}$,

$TiO_{4/2}$ oder $R'TiO_{3/2}$ oder $R'_2\ TiO_{2/2}$,

$ZrO_{4/2}$ oder $R'ZrO_{3/2}$ oder $R'_2\ ZrO_{2/2}$,

$AlO_{3/2}$ oder $R'AlO_{2/2}$

wobei R' eine Methyl- oder Ethylgruppe ist, und/oder durch Phenyleneinheiten der allgemeinen Formel

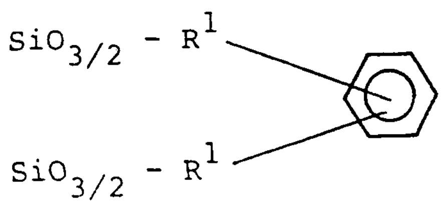

(2)

in der die Brückenglieder $R^1$ denselben Bedeutungsumfang wie bei Formel (1) haben und gleich oder verschieden sein können, abgesättigt sind und das Verhältnis der Summe der Si-Atome der Einheiten nach Formeln (1) und (2) zu den Brückenatomen Silicium, Titan, Zirkonium und Aluminium 1 : 0 bis 1 : 15 betragen kann.

**2.** Verfahren zur Herstellung der Verbindungen nach Anspruch 1, dadurch gekennzeichnet, daß ein Phenylengruppen-haltiges Organopolysiloxan, welches gleiche oder verschiedene Einheiten der allgemeinen Formel

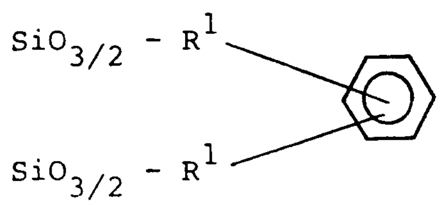

(2)

enthält, in der jeweils alle 3 denkbaren Isomeren in bezug auf die Stellung der $SiO_{3/2}$-$R^1$-Substituenten an der Phenylengruppe nebeneinander vorliegen können, wobei die Brückenglieder $R^1$ für die Gruppen $-CH_2-CH_2-$ oder $CH_3-CH<$ stehen und gleich oder verschieden sein können, und die freien Valenzen der Sauerstoffatome durch Siliciumatome weiterer Gruppen der Formel (2) und/oder durch vernetzende Brückenglieder

$SiO_{4/2}$ oder $R'SiO_{3/2}$ oder $R'_2\ SiO_{2/2}$,

$TiO_{4/2}$ oder $R'TiO_{3/2}$ oder $R'_2\ TiO_{2/2}$,

$ZrO_{4/2}$ oder $R'ZrO_{3/2}$ oder $R'_2\ ZrO_{2/2}$,

$AlO_{3/2}$ oder $R'AlO_{2/2}$

wobei R' eine Methyl- oder Ethylgruppe ist, abgesättigt sind und das Verhältnis der Summe der Si-Atome in Formel (2) zu den Brückenatomen Silicium, Titan, Zirkonium und Aluminium 1 : 0 bis 1 : 15 betragen kann, in einem Lösungsmittel, bei Temperaturen von -80 °C bis 200 ° C, bei Normaldruck

oder einem Überdruck, welcher der Summe der Partialdrucke der Komponenten der Reaktionsmischung bei der jeweiligen Temperatur entspricht, mit stöchiometrischen, unterschüssigen oder überschüssigen Mengen eines teilweise oder vollständig gelösten, gasförmigen, flüssigen oder festen Reagenzes für die an sich bekannte Substitution mindestens eines ringständigen Wasserstoffatoms durch den für eine beabsichtigte Anwendung erforderlichen funktionellen Substituenten X über einen Zeitraum von wenigen Minuten bis zu mehreren Tagen bei Temperaturen von -78° C bis 200° C umgesetzt wird, dieser Umsetzungsvorgang gegebenenfalls nach einem Wechsel des Lösungsmittels wiederholt, anschließend der Feststoff von der flüssigen Phase nach gängigen Techniken abgetrennt oder das Lösungsmittel destillativ entfernt wird, der modifizierte Feststoff gegebenenfalls unter Verwendung eines anderen Lösungsmittels gewaschen, dann gegebenenfalls unter Schutzgasatmosphäre oder unter Verwendung von Vakuum bis zu einer Temperatur von 200° C getrocknet, anschließend gegebenenfalls 1 Stunde bis zu 5 Tagen bei Temperaturen von 100 - 400° C an der Luft oder unter Schutzgas, bei Normaldruck, unter Vakuum oder Überdruck getempert, gegebenenfalls gemahlen und/oder klassifiziert wird.

3. Verfahren gemäß Anspruch 2, dadurch gekennzeichnet, daß als Funktionalisierungsagenzien elementares Chlor, Brom, Chlormethylmethyl- oder Chlormethylethylether sowie Formaldehyd, das Methyl- oder Ethylacetal von Formaldehyd, Paraformaldehyd jeweils in Kombination mit Chlorwasserstoff, und Lithium-, Natrium- oder Kaliumdiphenylphosphid verwendet wird.

## Claims

1. Functionalised organopolysiloxanes containing phenylene groups, characterised in that they contain identical or different units corresponding to the following formula

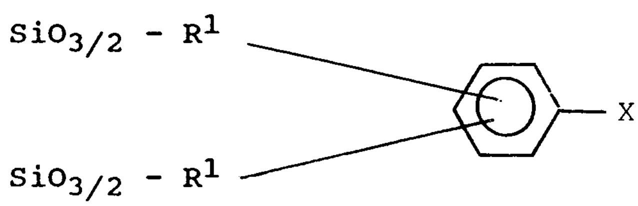

(1)

in which all 3 conceivable isomers with respect to the position of the $SiO_{3/2}R^1$ substituents on the phenylene group may be present side by side and in which the bridging members $R^1$ stand for the groups $-CH_2-CH_2-$ or $CH_3-CH<$ and may be identical or different,

X stands for Cl, Br, $CH_2Cl$, $P(C_6H_5)_2$ or $CH_2P(C_6H_5)_2$ and the free valencies of the oxygen atoms are saturated by silicon atoms of further groups corresponding to Formula (I) and/or by cross-linking bridging members

$SiO_{4/2}$ or $R'SiO_{3/2}$ or $R'_2\,SiO_{2/2}$,

$TiO_{4/2}$ or $R'TiO_{3/2}$ or $R'_2\,TiO_{2/2}$,

$ZrO_{4/2}$ or $R'ZrO_{3/2}$ or $R'_2\,ZrO_{2/2}$,

$AlO_{3/2}$ or $R'AlO_{2/2}$

wherein R' denotes a methyl or ethyl group, and/or by phenylene units corresponding to the general formula

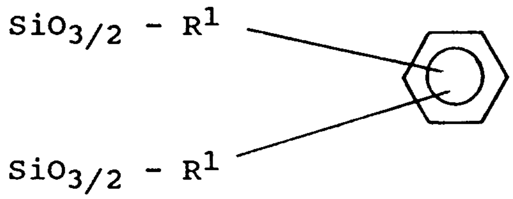

(2)

in which the bridging members $R^1$ have the same range of meanings as in Formula (1) and may be identical or different, and the ratio of the sum of Si atoms of the units corresponding to Formulae (1) and (2) to the bridging atoms, silicon, titanium, zirconium and aluminium may be from 1 : 0 to 1 : 15.

**2.** A process for the preparation of the compounds according to Claim 1, characterised in that an organopolysiloxane containing phenylene groups and containing identical or different units corresponding to the following general formula

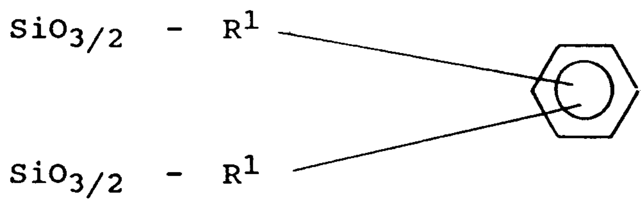

(2)

in which all 3 conceivable isomers with respect to the position of the $SiO_{3/2}$-$R^1$ substituents on the phenylene group may be present side by side and the bridging members $R^1$ stand for the groups $CH_2$-$CH_2$ or $CH_3$-$CH<$ and may be identical or different, and the free valencies of the oxygen atoms are saturated by silicon atoms of further groups of Formula (2) and/or by cross-linking bridging members,

$SiO_{4/2}$ or $R'SiO_{3/2}$ or $R'_2\ SiO_{2/2}$,

$TiO_{4/2}$ or $R'TiO_{3/2}$ or $R'_2\ TiO_{2/2}$,

$ZrO_{4/2}$ or $R'ZrO_{3/2}$ or $R'_2\ ZrO_{2/2}$,

$AlO_{3/2}$ or $R'AlO_{2/2}$

wherein R' denotes a methyl or ethyl group, and the ratio of the sum of Si atoms in Formula (2) to the bridging atoms, silicon, titanium, zirconium and aluminium may be from 1 : 0 to 1 : 15 is reacted in a solvent at temperatures from -80 °C to 200 °C and at normal pressure or at an excess pressure corresponding to the sum of the partial pressures of the components of the reaction mixture at the given temperature with stoichiometric, subequivalent or excess quantities of a partially or completely dissolved gaseous, liquid or solid reagent for the substitution known per se of at least one ring hydrogen atom by the functional substituents X required for an intended use, the reacton being carried out over a period from a few minutes to several days at temperatures of from -78 °C to 200 °C, this reaction procedure is optionally repeated after a change of solvent, and the solid is then separated from the liquid phase by conventional techniques or the solvent is removed by distillation, the modified solvent is optionally washed with a different solvent, and the product is then optionally dried under a protective gas atmosphere or under a vacuum up to a temperature of 200 °C and is then optionally tempered in air or under a protective gas at temperatures of from 100 to 400 °C for a period of from one to five days at normal pressure or under a vacuum or excess pressure and optionally ground and/or classified.

**3.** A process according to Claim 2, characterised in that the functionalizing agents used are elementary chlorine, bromine, chloromethyl methyl ether or chloromethyl ethyl ether, formaldehyde, the methyl or ethyl acetal of formaldehyde, paraformaldehyde, in each case in combination with hydrogen chloride, and the diphenylphosphide of lithium or of sodium or of potassium.

## Revendications

**1.** Organopolysiloxanes contenant des groupes phénylène fonctionnalisés, caractérisés en ce qu'ils contiennent des motifs, identiques ou différents, de formule

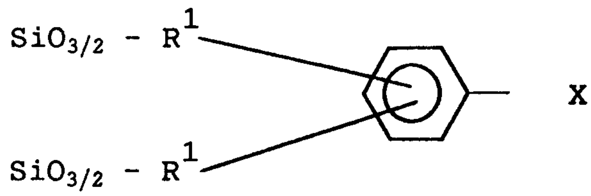

(1)

dans laquelle tous les 3 isomères envisageables en ce qui concerne la position des substituants $SiO_{3/2}$-$R^1$ sur le groupe phénylène peuvent être présents simultanément dans chaque cas, les chaînons pontants $R^1$représentant les groupes $-CH_2-CH_2-$ ou $CH_3-CH<$ et pouvant être identiques ou différents, X représente Cl, Br, $CH_2Cl$, $P(C_6H_5)_2$, $CH_2P(C_6H_5)_2$ et les valences libres des atomes d'oxygène sont saturées par des atomes de silicium d'autres groupes de formule (1) et/ou par les chaînons pontants réticulants
$SiO_{4/2}$ ou $R'SiO_{3/2}$ ou $R'_2$ $SiO_{2/2}$
$TiO_{4/2}$ ou $R'TiO_{3/2}$ ou $R'_2$ $TiO_{2/2}$
$ZrO_{4/2}$ ou $R'ZrO_{3/2}$ ou $R'_2$ $ZrO_{2/2}$
$AlO_{3/2}$ ou $R'AlO_{2/2}$
R' étant un groupe méthyle ou éthyle, et/ou par des motifs phénylène de formule générale

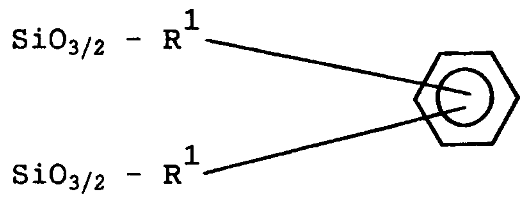

(2)

dans lesquels les chaînons pontants $R^1$ ont la même signification que dans la formule (1) et peuvent être identiques ou différents, et le rapport de la somme des atomes de Si des motifs de formules (1) et (2) aux atomes pontants silicium, titane, zirconium et aluminium peut aller de 1 : 0 à 1 : 15.

**2.** Procédé pour la préparation des composés selon la revendication 1, caractérisé en ce qu'un organopolysiloxane contenant des groupes phénylène, lequel contient des motifs, identiques ou différents, de formule générale

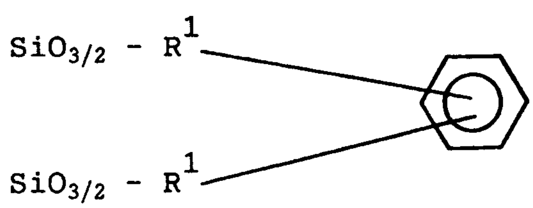

(2)

dans laquelle tous les 3 isomères envisageables, en ce qui concerne la position des substituants $SiO_{3/2}$ - $R^1$ sur le groupe phénylène peuvent être présents simultanément dans chaque cas, les chaînons pontants $R^1$ représentant les groupes $-CH_2-CH_2-$ ou $CH_3-CH<$ et pouvant être identiques ou différents et les valences libres des atomes d'oxygène étant saturées par des atomes de silicium d'autres groupes de formule (2) et/ou par les chaînons pontants réticulants
$SiO_{4/2}$ ou $R'SiO_{3/2}$ ou $R'_2$ $SiO_{2/2}$
$TiO_{4/2}$ ou $R'TiO_{3/2}$ ou $R'_2$ $TiO_{2/2}$
$ZrO_{4/2}$ ou $R'ZrO_{3/2}$ ou $R'_2$ $ZrO_{2/2}$

$AlO_{3/2}$ ou $R'AlO_{2/2}$

R' étant un groupe méthyle ou éthyle et le rapport de la somme des atomes de Si dans la formule (2) aux atomes pontants silicium, titane, zirconium et aluminium pouvant aller de 1 : 0 à 1 : 15, dans un solvant, à des températures de -80 à 200 ° C, à la pression normale ou sous une surpression qui correspond à la somme des pressions partielles des composants du mélange réactionnel à la température respective, avec des quantités stoechiométriques, plus faibles ou en excès, d'un réactif gazeux, liquide ou solide, dissous en partie ou en totalité, pour la substitution connue en soi d'au moins un atome d'hydrogène situé sur le noyau par le substituant fonctionnel X requis pour une application projetée sous un espace de temps de quelques minutes à plusieurs jours à une température comprise entre -78 ° C et 200 ° C, on répète éventuellement ce processus réactionnel après un changement du solvant, on sépare ensuite la matière solide de la phase liquide selon des techniques courantes, ou on élimine le solvant par distillation, on lave la matière solide modifiée, en utilisant éventuellement un autre solvant, puis on la sèche à une température allant jusqu'à 200 ° C, éventuellement sous une atmosphère de gaz inerte ou avec utilisation de vide, ensuite on l'étuve éventuellement pendant 1 heure à 5 jours, à des températures de 100 à 400 ° C, à l'air ou sous un gaz inerte, sous la pression normale, sous vide ou sous une surpression, éventuellement on la broie et/ou la classe par taille.

**3.** Procédé selon la revendication 2, caractérisé en ce que l'on utilise en tant que réactifs de fonctionnalisation du chlore ou du brome élémentaire, un éther chlorométhyl-méthylique ou chlorométhyléthylique, ainsi que le formaldéhyde, l'acétal méthylique ou éthylique du formaldéhyde, le paraformaldéhyde, chacun en combinaison avec de l'acide chlorhydrique et le diphénylphosphure de lithium, sodium ou potassium.